(19)

Europäisches
Patentamt
European
Patent Office
Office européen
des brevets

(11) **EP 2 496 275 B1**

(12) **EUROPEAN PATENT SPECIFICATION**

(45) Date of publication and mention
of the grant of the patent:
**20.05.2015 Bulletin 2015/21**

(21) Application number: **10773223.2**

(22) Date of filing: **28.10.2010**

(51) Int Cl.:
***A61L 15/56*** *(2006.01)*     ***A61L 15/60*** *(2006.01)*

(86) International application number:
**PCT/US2010/054367**

(87) International publication number:
**WO 2011/056689 (12.05.2011 Gazette 2011/19)**

(54) **ABSORBENT ARTICLE HAVING ACTIVATED COLOR REGIONS IN OVERLAPPING LAYERS**

SAUGFÄHIGER ARTIKEL MIT AKTIVIERTEN FARBIGEN ZONEN IN ÜBERLAPPENDEN LAGEN

ARTICLE ABSORBANT COMPRENANT DES RÉGIONS COLORÉES ACTIVÉES DANS DES COUCHES EN CHEVAUCHEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.11.2009 US 611962**

(43) Date of publication of application:
**12.09.2012 Bulletin 2012/37**

(73) Proprietor: **The Procter & Gamble Company
Cincinnati, OH 45202 (US)**

(72) Inventors:
• **ARORA, Kelyn, Anne
Cincinnati
Ohio 45226 (US)**

• **HAMMONS, John, Lee
Hamilton
Ohio 45011 (US)**

(74) Representative: **Kremer, Véronique Marie
Joséphine et al
Procter & Gamble Service GmbH
IP Department
Frankfurter Strasse 145
61476 Kronberg im Taunus (DE)**

(56) References cited:
**EP-A1- 1 295 711     WO-A1-2007/032711
WO-A1-2009/013659     WO-A1-2011/025486
WO-A2-2005/107670**

**Description**

FIELD OF THE INVENTION

The present invention is related to activatable colorants that are activated to produce color. Specifically, the invention is related to activatable colorants disposed on overlapping layers of materials that are activated to produce colored regions on the overlapping layers.

BACKGROUND OF THE INVENTION

A variety of absorbent articles that include different colored regions are available in the market. For instance, absorbent articles such as sanitary napkins and female adult incontinence articles that function to collect fluid discharged from a woman's vagina or urethra sometimes include a colored region proximal the central portion of the absorbent article that differs in color from portions of the absorbent article remote from the central portion of the absorbent article. Absorbent articles such as sanitary napkins have also been known to include decorative designs on the topsheet and backsheet that are appealing to consumers. However, absorbent articles currently available in the market place are generally provided with colored regions on only a single component, such as the secondary topsheet or topsheet or backsheet. One reason for limiting the colored regions to such single components is the difficulties associated with registering colored regions disposed on multiple components during manufacturing. Nevertheless, limiting colored regions to a single component or layer limits the design space in which designers can create innovative designs that meet consumer demands.

High speed manufacturing lines that include printing capability represent a high capital cost to manufacturers of absorbent articles. For manufacturers to effectively recover the cost of such capital, it is advantageous for manufactures to use existing manufacturing lines to continue manufacturing absorbent articles. In some instances, the approach manufacturers have chosen to provide for colored regions might not be easily adapted to provide for colored regions that are disposed on multiple components or layers due to the crowded nature of the manufacturing line. Thus, if a manufacturer desires to provide for visual elements on multiple components of the absorbent article, the manufacturer might have to retool the manufacturing line to provide for additional printing and registration capabilities, thus incurring additional capital cost.

With these limitations in mind, there is a continuing unaddressed need for absorbent articles that can be manufactured cost effectively using existing manufacturing capability that can be provided with colored regions on multiple layers so that designers have a richer palette of color impression with which to work. Still further there is a need for providing absorbent articles with colored regions on multiple layers without requiring additional printing capabilities for printing on multiple layers or registration capabilities for registering the colored regions on multiple layers during manufacturing.

WO2005107670 discloses absorbent articles for feminine hygiene performing color changes when exposed to an external stimulus.

SUMMARY OF THE INVENTION

The invention concerns a tampon/applicator assembly according to claim 1.

BRIEF DESCRIPTION OF THE DRAWINGS

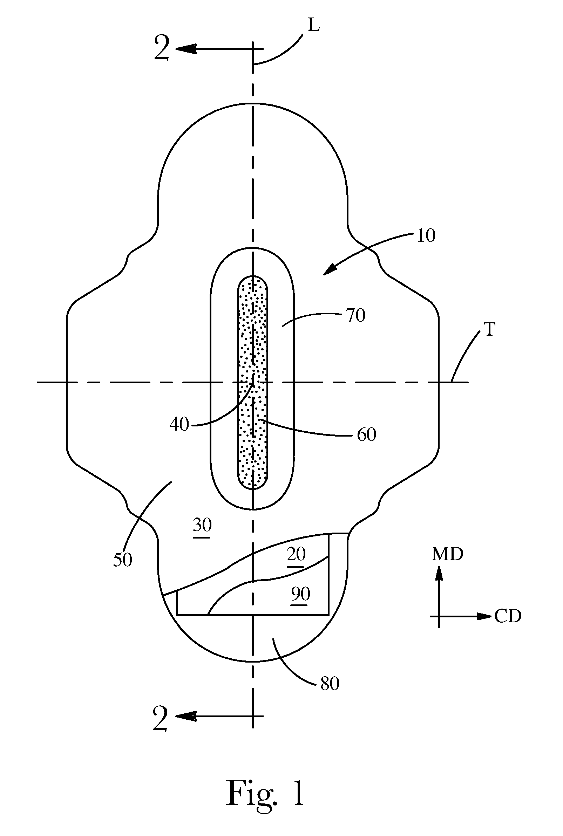

    FIG. 1 is a schematic of an absorbent article. (not falling under the present invention)

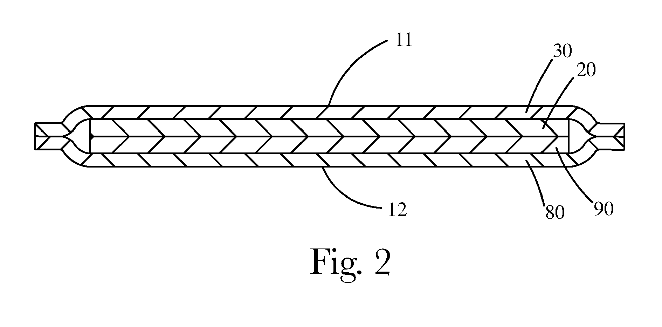

    FIG. 2 is a cross-section of the absorbent article illustrated in FIG. 1.

DETAILED DESCRIPTION OF THE INVENTION

Definitions:

As used herein and in the claims, the term "comprising" is inclusive or open-ended and does not exclude additional unrecited elements, compositional components, or method steps.

As used herein, the term "nonwoven web" refers to a web having a structure of individual fibers or threads which are interlaid, but not in a repeating pattern as in a woven or knitted fabric, which do not typically have randomly oriented fibers. Nonwoven webs or fabrics have been formed from many processes, such as, for example, meltblowing processes, spunbonding processes, hydroentangling, airlaid, and bonded carded web processes, including carded thermal bonding. The basis weight of nonwoven fabrics is usually expressed in grams per square meter (g/m2). The basis weight of a laminate web is the combined basis weight of the constituent layers and any other added components. Fiber diameters are usually expressed in microns; fiber size can also be expressed in denier, which is a unit of weight per length of fiber. The basis weight of laminate webs suitable for use in the present invention can range from 6 g/m2 to 400 g/m2, depending on the ultimate use of the web. For use as a hand towel, for example, both a first web and a second web can be a nonwoven web having a basis weight of between 18 g/m2 and 500 g/m2.

As used herein, the term "tampon" refers to any type of absorbent structure such as, e.g., an absorbent mass, that can be inserted into the vaginal canal or other body cavity, such as, e.g., for the absorption of fluid therefrom, to aid in wound healing, and/or for the delivery of materials, such as moisture or active materials such as medicaments. In general, the term "tampon" is used to refer to a finished tampon after the compression and/or shaping process.

As used herein, the term "applicator" refers to a device or implement that facilitates the insertion of a feminine hygiene product, such as, e.g., a tampon or pessary, into an external orifice of a mammal. Suitable applicators include, e.g., telescoping, tube and plunger, and compact applicators.

The term "color" as referred to herein includes any primary color, i.e., white, black, red, blue, violet, orange, yellow, green, and indigo as well as any declination thereof or mixture thereof. The term 'non-color' or 'non-colored' refers to the color white which is further defined as those colors having an L* value of at least 90, an a* value equal to 0 ± 2, and a b* value equal to 0±2.

"Color change" herein means that at least a part of layer including an activatable colorant changes its color in response to an external stimulus. The change in color is visible from outside the layer. A change in color "visible from outside the layer" as used herein means that the color change is detectable by the naked human eye.

"Activatable colorant" means a material which provides the color change in response to an external stimulus.

"External stimulus" means the exposure of the absorbent article to energy from outside the article in the form of light

"Activated color region" means areas containing a colorant that has been activated by external stimulus.

"Visible" means those colors and wavelengths of light that are detectable by the human eye, nominally about 400-700 nanometers in wavelength.

"Electromagnetic radiation" means those areas of the spectrum amenable to industrial applications, such as the ultraviolet through the infrared wavelengths

"Activatable chemistry" means those chemicals, monomers and polymers which are capable of being affected by an external stimulus.

The activatable colorant can produce a color change that is reversible or irreversible. However, preferably the activatable colorant according to the present invention produces a color change that is irreversible, thereby providing a permanent visual effect. Sources of activatable colorants include 'photoreactive', which means that the color change is induced by electromagnetic radiation, discussed more fully below.

The overlapping layers according to the present invention include films, nonwovens, air laids, fibers, filaments, adhesives, lotions, absorbent gelling materials and foams. The composition used to form the overlapping layers of the present invention, particularly films and nonwovens, can include thermoplastic polymeric and non-thermoplastic polymeric materials. For fibers and nonwovens, thermoplastic polymeric material used in forming fibers must have rheological characteristics suitable for melt spinning. The molecular weight of the polymer must be sufficient to enable entanglement between polymer molecules and yet low enough to be melt spinnable. For melt spinning, thermoplastic polymers have molecular weights below about 1,000,000 g/mol, preferably from about 5,000 g/mol to about 750,000 g/mol, more preferably from about 10,000 g/mol to about 500,000 g/mol and even more preferably from about 50,000 g/mol to about 400,000 g/mol. Unless specified elsewhere, the molecular weight indicated is the number average molecular weight.

The thermoplastic polymeric materials are able to solidify relatively rapidly, preferably under extensional flow, and form a thermally stable fiber structure, as typically encountered in known processes such as a spin draw process for staple fibers or a spunbond continuous fiber process. Preferred polymeric materials include, but are not limited to, polypropylene and polypropylene copolymers, polyethylene and polyethylene copolymers, polyester and polyester copolymers, polyamide, polyimide, polylactic acid, polyhydroxyalkanoate, polyvinyl alcohol, ethylene vinyl alcohol, polyacrylates, and copolymers thereof and mixtures thereof. Other suitable polymeric materials include thermoplastic starch compositions as described in detail in U.S. publications 2003/0109605A1 and 2003/0091803. Other suitable polymeric materials include ethylene acrylic acid, polyolefin carboxylic acid copolymers, and combinations thereof. Other suitable polymeric materials comprising starch and polymers are described in US publications 6,746,766, US 6,818,295, US 6,946,506 and US application 03/0092343. Common thermoplastic polymer fiber grade materials are preferred, most notably polyester based resins, polypropylene based resins, polylactic acid based resin, polyhydroxyalkonoate based resin, and polyethylene based resin and combination thereof. Most preferred are polyester and polypropylene based resins.

The overlapping layers according to the present invention can include an adhesive having an activated colorant incorporated in a component forming the adhesive. The adhesive can provide one of the two overlapping layers as a coating. Alternatively, the adhesive can be incorporated into a layer such as a nonwoven forming one of the two overlapping layers. Such an adhesive can comprise a hot melt adhesive.

Hot-melt adhesives used as construction adhesives in the manufacture of disposable absorbent articles typically include several components. These components include one or more polymers to provide cohesive strength, such as ethylene-vinyl acetate, copolymers, polypropylene, phenoxy resins, styrene-butadiene copolymers, ethylene-ethyl acrylate copolymers, low density polypropylenes, polyesters, polyamides, and polyurethanes. These polymers make up a significant part of the hot-melt adhesive composition. The composition also includes components such as, for example, a resin or analogous material (sometimes called a tackifier) to provide adhesive strength. Examples of such materials include hydrocarbons distilled from petroleum distillates, rosins and/or rosin esters, and terpenes derived, for example, from wood or citrus. The composition also typically includes waxes, plasticizers or other materials to modify viscosity. Examples of such materials include mineral oil, polybutene, paraffin oils, ester oils, and the like. Still further, the composition can optionally include additives, such as antioxidants or other stabilizers. A typical hot-melt adhesive composition might contain from about 15 to about 35 weight percent (wt.%) cohesive strength polymer(s); from about 50 to about 65 wt.% resin or other tackifier(s); from more than zero to about 30 wt.% plasticizer or other viscosity modifier; and optionally less than about 1 wt.% stabilizer or other additive.

For each of the aforementioned embodiments, the activatable colorant is blended into or coated onto material forming a layer. The activatable colorants are subsequently activated to change its color by electromagnetic radiation (exposure to ultraviolet,)

The activation occurs after the overlapping layers are put together in order to avoid having to register the color patterns during assembly. The activatable colorants disposed on the overlapping layers are activated simultaneously.

Overlapping layers according to the present invention comprise the same type of activatable colorant. The activatable colorant on the first layer and the second layer comprise a photoreactive material.

COLOR CHANGE MATERIAL

As briefly described above, the color change material is 'photoreactive', which means that the color change is induced by electromagnetic radiation. These definitions comprise materials changing color irreversibly, reversibly or quasi-reversibly in response to the respective stimulus. The color change materials herein can either be coated onto parts of the absorbent article, such as on films or fibers, or can form an integral part of components of the absorbent article by being added e.g. to the polymeric master batch these components are made of. The color change materials herein change their color in response to external stimuli as defined hereinbefore.

c) Photoreactive Materials

Photoreactive materials change color in response to exposure to electromagnetic radiation. The color change can be irreversible providing a permanent change in color or it can be reversible providing a temporary change in color.

Photochromic materials are those that reversibly change color when exposed to light or changes in light intensity. Photochromic materials typically provide a reversible color change transiting from a colorless state to a color state upon exposure to light and back to a colorless state when reversed. Examples for photochromic materials are described in U.S. Pat. No. 6,306,409; U.S. Pat. No. 6,080,415 or U.S. Pat. No. 5,730,961.

Polychromic materials are those which are capable of generating multiple colors. Compounds based upon diacetylene, X-C≡C-C≡C-Y, when polymerized, are known to take on different color properties. Polymerization is typically achieved by exposure to certain types of radiation, such as ultraviolet radiation. Varying the intensity of the radiation causes differing degrees of polymerization, and different colors.

It is known that these properties can be utilized to achieve multi-color printing. See, for example; U.S. Pat. No. 4,705,742, "Processless Multicolour Imaging", issued on Nov. 10, 1987, assigned to Gaf Corporation; and WO2006/018640, "Multi-colour printing", published on Feb. 23, 2006, Sherwood Technologies Ltd. Both of these documents disclose methods of applying coatings comprising various diacetylene compounds to the surface of a substrate for the purpose of irradiating and forming an image on the surface of the substrate.

Particularly preferred materials are those that can be dispersed or blended into the polymeric matrix of the layers, such as those disclosed in PCT publication WO 2009/093028A2 and WO 2009/081385 A2, which are compounds which undergo a color change upon irradiation, and which have the general structure: X-C≡ C-C≡ C-Y-(CO)n-QZ wherein X is H, alkyl or -Y-(CO)n-QW; each Y is the same or a different divalent alkylene group; Q is O, S or NR; R is H or alkyl; W is H, alkyl or Z; each Z is the same or a different unsaturated alkyl group; and each n is 0 or 1.

Another example of a material of use in the present invention is a thermoplastic material comprising polymer mixed with a charge transfer agent and a photo acid generating agent such as those described in US 2009/0191476 A1. Exposure of the thermoplastic material comprising the charge transfer agent and photo acid generating agent to irradiation will bring about a color change reaction which can be used to create text, artwork, devices or other images and effects.

Absorbent articles according to the present invention preferably comprise photoreactive materials providing an irreversible, permanent change in color. Examples of photoreactive materials providing permanent color change are described in PCT publication WO 2009093028A2 which describes polychromic substances comprising diacetylene compounds that change color when subjected to irradiation. The type of radiation that performs the color change reaction with the diacetylene compounds includes laser or non-coherent, broadband or monochromatic radiation. Radiation type is ultraviolet.

Ultraviolet irradiation is preferred for changing substrates comprising the diacetylene compounds from colorless or low visual color to color on exposure to ultraviolet irradiation, and then change to a color different to the first on subsequent exposure to infrared irradiation. Laser irradiation may be preferred for writing text and drawing intricate artwork directly on substrates comprising the diacetylene compounds, as laser imaging can be conveniently controlled by computer with the appropriate software and has superior resolution capability. However, similar effects can be obtained by passing radiation from, for example, an ultraviolet lamp through a mask before it reaches the substrates comprising the diacetylene compound.

Another application describing of photoreactive materials providing permanent color change includes WO 2009/081385 which describes thermoplastic material comprising polychromic substance wherein the polychromic substance is a functionalized diacetylene having a formula which has a general structure that is described therein.

Activation of photoreactive materials is preferably achieved using an ultraviolet lamp. One example is the Coil Clean (CC) Series ultraviolet fixtures available from American Ultraviolet (Lebanon, IN). Another UVC exposure unit suitable for use in activation of photoreactive materials consists of a metal enclosure containing 8 UV amalgam lamps and 8 ballasts with individual circuits for individual lamp controls and a fan for cooling lamps to maintain temperature. The lamps are 357 mm in length and are available from American Ultraviolet as part number GML750A.

Other examples of equipment that may be used for activation of photoreactive materials include the J3825 MonoCure Lamphead from Nordson UV Limited (Berkshire UK) and the 270S UV Lamp Assembly and Power Supply by Integrated Technology. The type of lamp within the unit may be changed to vary the spectral output as needed. Examples of relevant bulb types include "H", "V", "D" and "Q".

The absorbent article embodiments including overlapping layers with activatable colorants include a tampon and an applicator where the first layer comprises the tampon and the second overlapping layer comprises the applicator.

The present invention is applicable to a tampon and applicator combination.

Providing different colored regions on different layers of material of the absorbent article 5 can create a richer visual impression on the absorbent article 5. For instance, if the first activated color region 60 and second activated color region 70 are on different layers of materials, when viewed, at least one of the colored regions will be viewed through the layer comprising the other colored region. A colored region viewed through another layer material can have a significantly different visual impression in terms of softness/diffuseness of the image, somewhat like the difference between a matte finished photograph versus a gloss finished photograph or the way an undergarment looks beneath a sheer article of clothing. Further, if the first layer 20 and second layer 22 are different material types, for example one is a film and the other is a nonwoven, different concentrations of the same activatable colorant might be used on each layer. Alternatively, a top layer and lower layer may both contain photoreactive colorants, but the lower layer may have a higher concentration in order to achieve the same or darker color for color matching or depth perception, respectively.

To provide for more visually coherent designs, the first activated colored region 60 and second activated colored region 70 can be within a CIELab color space volume of less than about 200. CIELab color space volume is discussed in more detail below. With such an approach, the colors of the first activated colored region 60 and second activated colored region 70 do not differ substantially to the eye of most viewers and viewers might perceive the colors to be shades or subtle variations of the same color. Subtle variations in color are thought to be pleasing to the eye, much like sample paint chips having slightly varying colors found in home decoration stores that can be pleasurable and interesting to view. If less distinctiveness between the first activated colored region 60 and second activated colored region 70 is desired, the first activated colored region 60 and second activated colored region 70 can be within a CIELab color space volume of less than about 50.

The color of the first activated colored region 60 and second activated colored region 70 and background region 50 are measured by the reflectance spectrophotometer according to the colors L*, a*, and b* values. The L*, a*, and b* values are measured from the body facing surface 10 of the absorbent article 5 inboard of the periphery 110 of the absorbent article 5. The difference in color is calculated using the L*, a*, and b* values by the formula $\Delta E = [(L*_X.-L*_Y)2 + (a*_X. - a*_Y)2 + (b*_X - b*_Y)2]1/2$. Herein, the 'X' in the equation may represent the first activated colored region 60, the second activated colored region 70, or the background region 50 and 'Y' may represent the color of another region against which the color of such region is compared. X and Y should not be the same two points of measurement at the same time. In other words, for any particular comparison of the difference in color, the location of X does not equal ($\neq$) the location of Y.

Where more than two colors are used, the 'X' and 'Y' values alternately include points of measurement in them also. The key to the $\Delta E$ calculation herein is that the 'X' and 'Y' values should not stem from the same measured point on the viewing surface. In those instances where there is effectively no non-colored portion 50 within the confines of the measurement area, the 'X' values should flow from a point different in spatial relationship to the 'Y' values, but within the confines of the absorbent core periphery.

Reflectance color is measured using the Hunter Lab LabScan XE reflectance spectrophotometer obtained from Hunter Associates Laboratory of Reston, Va. An absorbent article 5 is tested at an ambient temperature between 65 oF and 75 oF and a relative humidity between 50% and 80%.

The spectrophotometer is set to the CIELab color scale and with a D50 illumination. The Observer is set at 10° and the Mode is set at 45/0°. Area View is set to 0.125" and Port Size is set to 0.20" for films; Area View is set to 1.00" and Port Size is set to 1.20" for nonwovens and other materials. The spectrophotometer is calibrated prior to sample analysis utilizing the black and white reference tiles supplied from the vendor with the instrument. Calibration is done according to the manufacturer's instructions as set forth in LabScan XE User's Manual, Manual Version 1.1, August 2001, A60-1010-862. If cleaning is required of the reference tiles or samples, only tissues that do not contain embossing, lotion, or brighteners should be used (e.g., PUFFS tissue). Any sample point on the absorbent article containing the activated color to be analyzed can be selected.

The absorbent article 5 is placed over the sample port of the spectrophotometer with a white tile placed behind the absorbent article 5. The absorbent article 5 is to be in a substantially flat condition and free of wrinkles.

The absorbent article 5 is removed and repositioned so that a minimum of six readings of color of the body facing surface 10 are conducted. If possible (e.g., the size of the activated color on the element in question does not limit the ability to have six discretely different, nonoverlapping sample points), each of the readings is to be performed at a substantially different region on the externally visible surface so that no two sample points overlap. If the size of the activated colored region requires overlapping of sample points, only six samples should be taken with the sample points selected to minimize overlap between any two sample points. The readings are averaged to yield the reported L*, a*, and b* values for a specified color on an externally visible surface of an element.

In calculating the CIELab color space volume, V, maximum and minimum L*, a*, and b* values reported are determined for a particular set of regions to be measured. The maximum and minimum L*, a*, and b* values reported are used to calculate the CIELab color space volume, V according to the following formula:

$$V = \frac{4}{3}\left|\frac{\Delta L^*}{2}\right|\left\|\frac{\Delta a^*}{2}\right\|\left\|\frac{\Delta b^*}{2}\right|$$

Within the above formula, ΔL* is the difference in L* values between the two colored regions being compared and is calculated by: ΔL*=L*X-L*Y. The Δa* is the difference in a* values between the two colored regions being compared and is calculated by: Δa*=a*X-a*Y. The Δb* is the difference in b* values between the two colored regions being compared and is calculated by: Δb*=b*X-b*Y. The CIELab color space volume can result in a solid substantially ellipsoidal in shape. If ΔL*, Δa*, and Δb* are equal, the solid will be spherical. As used herein, a "solid" refers to the mathematical concept of a three-dimensional figure having length, breadth, and height (or depth). An ellipsoidal volume is preferred to calculate volume because an ellipsoid generally requires the dimensional differences of ΔL*, Δa*, and Δb* to be relatively more uniform than other solids. Furthermore, it is believed that ellipsoidal volumes are more visually acceptable (i.e., less detectable color mismatch by human perception) than spherical volumes.

In some embodiments, the activated colors of at least two externally visible surfaces of discrete elements will occupy a CIELab color space volume of less than about 200. The externally visible surfaces are analyzed according to the Test Method described below. Upon analysis, the inherent color of an element comprising an externally visible surface will yield L*, a*, and b* coordinates. The CIELab color space volume is then calculated using the formula presented above. The resulting volume can be less than about 200. The resulting volume can be less than about 50.

It should be recognized that the activated colors of more than two discrete colored regions may occupy the aforementioned CIELab color space volumes. In calculating the CIELab color space volume for more than two elements, the CIELab color space volume is calculated using the maximum and minimum L*, a*, and b* from a set of elements. The maximum color values and minimum color values are used to calculate V according to the formula presented above.

Example 2

A tampon comprising a nonwoven overwrap comprising a photoreactive colorant is inserted into a translucent plastic applicator comprising the same photoreactive colorant during the converting process. The tampon/applicator assembly is subsequently exposed to UV light through a patterned mask to create a color zone on both the applicator and the tampon. These color zones are perfectly registered with one another and work together to create and highlight a visual signal to the consumer of enhanced absorbency.

**Claims**

1. A tampon/applicator assembly comprising:

(a) a tampon comprising a nonwoven overwrap comprising a photoreactive colorant in a UV-activated color zone of said nonwoven overwrap, wherein said UV-activated color zone is in the shape of a patterned mask; and
(b) a translucent plastic applicator comprising the same photoreactive colorant in a UV-activated color zone of said translucent plastic applicator, wherein said UV-activated color zone is in the shape of the said patterned mask;

wherein said color zones are in perfect registration with one another and work together to create a visual signal to a consumer of enhanced absorbency.

**Patentansprüche**

1. Tampon/Applikator-Einheit, umfassend:

(a) einen Tampon, umfassend eine Vliesumwicklung, umfassend einen fotoreaktiven Farbstoff in einer UV-aktivierten Farbzone der Vliesumwicklung, wobei die UV-aktivierte Farbzone in Form einer gemusterten Maske vorliegt; und
(b) einen lichtdurchlässigen Kunststoffapplikator, umfassend denselben fotoreaktiven Farbstoff in einer UV-aktivierten Farbzone des lichtdurchlässigen Kunststoffapplikators, wobei die UV-aktivierte Farbzone in Form der gemusterten Maske vorliegt;

wobei die Farbzonen in perfekter Passgenauigkeit miteinander vorliegen und zusammenwirken, um ein optisches Signal von erhöhtem Absorptionsvermögen für einen Verbraucher zu erzeugen.

**Revendications**

1. Ensemble tampon/applicateur comprenant :

(a) un tampon comprenant un recouvrement non tissé comprenant un colorant photoréactif dans une zone de couleur activée par UV dudit recouvrement non tissé, dans lequel ladite zone de couleur activée par UV est sous la forme d'un masque à dessins ; et
(b) un applicateur en plastique translucide comprenant le même colorant photoréactif dans une zone de couleur activée par UV dudit applicateur en plastique translucide, dans lequel ladite zone de couleur activée par UV est sous la forme dudit masque à dessins ;

dans lequel lesdites zones de couleur sont en alignement parfait les unes avec les autres et travaillent ensemble pour créer un signal visuel d'absorbance améliorée pour un consommateur.

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005107670 A
- US 20030109605 A1
- US 20030091803 A1
- US 6746766 B
- US 6818295 B
- US 6946506 B
- US 030092343 B
- US 6306409 B

- US 6080415 A
- US 5730961 A
- US 4705742 A
- WO 2006018640 A
- WO 2009093028 A2
- WO 2009081385 A2
- US 20090191476 A1
- WO 2009081385 A

### Non-patent literature cited in the description

- LabScan XE User's Manual, Manual Version 1.1. August 2001, A60-1010, 862